# EUROPÄISCHE PATENTSCHRIFT

(11) **EP 1 501 585 B1**
(45) Veröffentlichungstag und Bekanntmachung des Hinweises auf die Patenterteilung: **11.03.2009**
(21) Anmeldenummer: 03717308.5
(22) Anmeldetag: 17.04.2003
(51) Int. Cl.: A61M 39/24, A61M 39/26

(54) **EINWEG-VENTILEINRICHTUNG**
ONE-WAY VALVE DEVICE
SYSTEME DE SOUPAPE UNIDIRECTIONNEL

(30) Priorität: 03.05.2002 DE 10219994
(43) Veröffentlichungstag der Anmeldung: 02.02.2005
(73) Patentinhaber: Filtertek B.V., County of Limerick (IE)
(72) Erfinder: MIJERS, Jan, Willem, Marinus, NL-2101 EH Heemstede (NL)
(74) Vertreter: Brose, D. Karl
(86) Internationale Anmeldenummer: PCT/EP2003/004017
(87) Internationale Veröffentlichungsnummer: WO 2003/092788

(56) Entgegenhaltungen:
- EP-A- 0 812 596
- DE-C- 19 643 360
- DE-C- 19 749 562
- US-A- 5 771 935

## Beschreibung

Die Erfindung betrifft eine Einweg-Ventileinrichtung, insbesondere ein Niederdruck-Rückschlagventil zu Verwendung für ein Infusionsbesteck, bestehend aus einem Gehäuse mit einem Zuleitungs- und einem Ableitungsrohr und mit zumindest einer in einem Druckraum einliegenden Dichtung.

Ventileinrichtungen der gattungsgemäßen Art zur Erzeugung eines Leerlaufstops werden bevorzugt für Infusionsbestecke in Krankenhäusern eingesetzt, wobei die Ventileinrichtung dafür verwendet werden, dass beim Leerlaufen eines mit Infusionsflüssigkeit gefüllten Vorratsbehälters ein sofortiger Leerlaufstop eintritt, damit keine Luft in die Infusionsflüssigkeit und gegen Ende des Zustroms über die vorhandenen Leitungen in das Venensystem eines Patienten gelangt. Eine Ventileinrichtung zur Verwendung in einem Infusionsbesteck ist beispielsweise aus der deutschen Offenlegungsschrift DE 29 19 343 A1 bekannt, welches mit einer Tropfkammer und einem Schwimmerventil ausgestattet ist, das bei leerlaufender Kammer das Eintreten von Luft in den Infusionsschlauch verhindert, wobei ausgangsseitig in der zur Infusionsnadel führenden flexiblen Rohrleitung eine Rollenklemme angeordnet ist. Zur Verhinderung einer Auffüllung des gesamten Leitungssystems des Dialysators mit Luft wird ein Doppelsitz-Schwimmerventil mit einer Schwimmerkugel verwendet. Derartige Schwimmerkugel-Ventile sprechen jedoch nicht immer exakt an, sodass ein Luftzutritt in den Infusionsschlauch nicht in jedem Fall verhindert werden kann.

Eine weitere Leerlaufsicherung ist aus der deutschen Offenlegungsschrift DE 36 32 412 A1 bekannt, bei der vor einer Tropfkammer ein Leerlaufventil in Strömungsrichtung angeordnet ist. Eine Schwimmerkugel liegt unter Reibung beim Abdichten an der Kanalwandung an und kann bei entsprechendem Bewegungsspiel eine Luftzufuhr nicht mit absoluter Sicherheit verhindern. Beide vorgenannten Lösungen sind nicht geeignet eine Luftzufuhr in das menschliche Venensystem auszuschließen.

Aus der DE 197 49 562 A1 ist ferner ein Infusionsbesteck bekannt, dass aus einem hochaufgehängten Behälter für die Infusionsflüssigkeit sowie einem an einem Drosselbehälter mittels eines Dornrohres anschließbaren Tropf, einer darunter auf einer flexiblen Zuleitung einstellbaren Rollenklemme und einer am Leitungsende befindlichen Injektionsnadel besteht. Um das Eindringen von Luft in die Infusionsflüssigkeit, insbesondere gegen Ende des Zustroms der Infusionsflüssigkeit zu verhindern, wird in Strömungsrichtung vor der Tropfkammer oder an deren Eingangsöffnung ein Leerlauf-Stop in die Strömung vorgeschaltet, mittels dessen eingebautem Ventils abhängig von einem anstehenden statischen Druck der Durchströmung die Infusionsflüssigkeit abstoppbar ist. Im praktischen Einsatz hat sich jedoch gezeigt, dass ein Leerlauf-Stop in Abhängigkeit des statisch anstehenden Drucks der Infusionsflüssigkeit zur Verhinderung des Eindringens von Luft nicht ausreichend ist. Insbesondere dann, wenn die Möglichkeit eines Vertauschens der Anschlüsse durch Unachtsamkeit gegeben und somit die Funktion des Leerlauf-Stop-Ventils nicht mehr gewährleistet ist. Dies wird besonders gefährlich, wenn Luft in das System eindringt, die beim Patienten, falls die Luft bis in die Venen vordringen, zu einer Embolie führen kann. Es ist daher wichtig, dass zusammen mit der Infusionsflüssigkeit keine Luft mitgeführt wird, die zu dem vorgenannten Nachteil führt. Ein besonderer Nachteil besteht darin, dass das vorbekannte Ventil im Falle des Anstehens eines statischen Überdrucks ausgangsseitig das Eindringen von Luft in das System nicht verhindern kann.

Der Erfindung liegt die Aufgabe zu Grunde, ein neuartiges Rückschlagventil mit niedrigem Öffnungsdruck aufzuzeigen, welches für ein Infusionsbesteck mit Gravitationsdruck und/oder Pumpendruck verwendbar ist und das Eindringen von Luft in das System des Infusionsbestecks mit höherer Sicherheit verhindert.

Erfindungsgemäß ist zur Lösung der Aufgabe vorgesehen, dass durch einen an den Zuleitungs- und/oder Ableitungsrohr anstehenden statischen Unterdruck oder ein Überdruck an dem Ableitungsrohr eine Unterbrechung des Durchflusses erfolgt. Das Eindringen von Luft in das System des Infusionsbestecks wird erfindungsgemäß dem zufolge in vorteilhafter Weise sowohl bei dem Anstehen eines statischen Unterdrucks als auch eines Überdrucks an dem Ableitungsrohr vermieden. Ein statischer Unterdruck an dem Zuleitungsrohr kann beispielsweise dann entstehen, wenn ein Vorratsbehälter der Infusionsflüssigkeit leergelaufen ist, wenn eine Infusionspumpe fehlerhaft arbeitet oder Luft in das System eingetreten ist. Ein Überdruck an dem Ableitungsrohr kann beispielsweise bei einem vorübergehenden Verschluss der Armvenen des Patienten auftreten, wenn ausgangsartig eine Medikamentenpumpe zusätzlich angeschlossen ist. Ein Überdruck würde dazu führen, dass das Medikament in das Schwerkraft-Infusionsbesteck gepumpt wird.

Durch die erfindungsgemäße Ventileinrichtung wird jedoch sichergestellt, dass in den vorgenannten Fällen ein weiterer Transport der Infusionsflüssigkeit zuverlässig gestoppt wird und somit das Eindringen von mit Luft angereicherter Infusionsflüssigkeit in die Venen verhindert wird. Zusätzlich wird durch das Einwege-Ventil sichergestellt, dass ausgangsseitig bei anstehenden Überdruck durch eine Infusions- oder Medikamentenpumpe das Ventil schließt und somit das Medikament nicht in das Infusionsbesteck gelangt, wodurch eine kurzzeitige Überdosierung verhindert wird. Zur Abdichtung des Zuleitungs- und Ableitungsrohres ist hierbei eine Dichtung vorgesehen, welche innerhalb eines Druckraumes auf einer Seite an einer ersten Dichtringlippe und auf der gegenüberliegenden Seite an einer zweiten Dichtringlippe wechselseitig jeweils unter Spannung anliegt. Alternativ besteht die Möglichkeit, dass zwei über einen Verbindungskanal kommunizierende Druckkammern ausgebildet sind, in welchen zumindest eine einstückige oder zwei getrennte Dichtungen an einer ersten und zweiten Dichtringlippe unter Spannung anliegen, wobei die beiden Dichtringlippen wiederum zu beiden Seiten der Dichtungen angeordnet sind oder der Verbindungskanal so ausgeführt ist, dass er einmal unterhalb und einmal oberhalb der Dichtung mündet. Mittels der Dichtungen erfolgt eine Abdichtung der Dichtringlippen, welche in Wirkverbindung mit dem Zuleitungs- bzw. Ableitungsrohr stehen und im Falle des Anliegens eines Unterdrucks eine Abdichtung gegenüber den Dichtringlippen ermöglichen. Aufgrund der wechselseitigen Abdichtung der Dichtringlippen durch zumindest eine Dichtung ist sichergestellt, dass im Falle eines Überdrucks an dem Ableitungsrohr nur eine Anhebung der Dichtung an der dem Ableitungsrohr zugeordnete Dichtringlippe stattfindet, während hingegen die Dichtung auf die zweite Dichtringlippe aufgrund des vorherrschenden Überdrucks gepresst wird und somit ein Verschluss des Zuleitungsrohres erfolgt.

In weiterer Ausgestaltung der Erfindung ist vorgesehen, dass das Zuleitungsrohr in eine Vorkammer mündet, in der die erste Dichtringlippe mit einer geschlossenen Mulde ausgebildet ist, an der eine den Druckraum begrenzende Dichtung anliegt, während das Ableitungsrohr innerhalb der zweiten Dichtringlippe in die Druckkammer des Gehäuses mündet, an der eine den Druckraum begrenzende Dichtung anliegt, wobei gegenüber der zweiten Dichtringlippe eine zweite durch die Dichtung begrenzende Vorkammer ausgebildet ist, welche durch eine Gehäuseöffnung mit Atmosphärendruck beaufschlagt ist. Durch das Zuleitungsrohr kann die Infusionsflüssigkeit in die Vorkammer einströmen und die an der ersten Dichtringlippe anliegende Dichtung anheben, in welche in weiterer Ausgestaltung der Erfindung im Bereich der ersten Dichtringlippe ein Öffnung vorhanden ist, welche zentrisch und innerhalb der ersten Dichtringlippe liegend ausgebildet ist, sodass der Druckraum gegenüber der Vorkammer abgedichtet ist. Die einströmende Infusionsflüssigkeit hebt diese Dichtung von der Dichtringlippe ab und kann durch die vorhandene Öffnung in die Druckkammer des Gehäuses einströmen, wobei durch die gegenüberliegende Anordnung der Dichtringlippe aufgrund des bestehenden Überdrucks die vorhandene einteilige oder zweiteilige Dichtung von der zweiten Dichtringlippe abgehoben wird, sodass eine Verbindung zum Ableitungsrohr entsteht. Hierbei handelt es sich um den normalen Strömungsvorgang der Infusionsflüssigkeit für den Fall, dass ein ausreichender statischer Überdruck am Zuleitungsrohr ansteht. Sollte hingegen am Zuleitungsrohr ein Unterdruck anliegen, verschließt die vorhandene Dichtung die erste Dichtringlippe durch den Ansaugeffekt und damit wird eine Unterbrechung des Strömungsflusses bewirkt. Ebenso verhält es sich, wenn an dem Ableitungsrohr ein Unterdruck anstehen sollte, da die Dichtung aufgrund einer vorhandenen Gehäuseöffnung im Bereich der ersten Dichtringlippe mit Atmosphärendruck beaufschlagt ist. Für den Fall, dass am Ableitungsrohr ein Überdruck entstehen sollte gelangt dieser über die zweite Dichtringlippe und die abhebende Dichtung nur bis in den Druckraum und presst somit die vorhandene Dichtung aufgrund des bestehenden Überdrucks unmittelbar auf die erste Dichtringlippe, sodass ein Strömungsfluss von dem Zuleitungsrohr zum Ableitungsrohr verhindert wird.

In weiterer Ausgestaltung der Erfindung ist vorgesehen, dass die von der ersten und zweiten Dichtringlippe eingeschlossene Fläche kleiner als die verbleibende Fläche der anliegende Dichtung ausgebildet ist und dass durch das Verhältnis der eingeschlossenen Fläche innerhalb der Dichtringlippe zur verbleibenden Fläche der anliegenden Dichtung der Öffnung- bzw. Schließdruck einstellbar ist. Für beide ausgebildeten Druckräume ist jeweils eine, vorzugsweise einteilige Dichtung, vorgesehen. Alternativ besteht die Möglichkeit, eine zweiteilige Dichtung zu verwenden, die beispielsweise bei zwei in sich geschlossenen Druckräumen eingesetzt wird, wobei die beiden Druckräume durch ein Verbindungskanal miteinander verbunden sind.

In weiterer Ausgestaltung der Erfindung ist vorgesehen, dass die Dichtung randseitig mit einem vorzugsweise T-förmigen Ansatz versehen ist, der in einer Ausnehmung des Gehäuses zu liegen kommt und einstückig mit der Dichtung ausgebildet ist. Durch den T-förmigen Ansatz wird eine einfache Befestigungsmöglichkeit der einen oder ggf. beider Dichtungen innerhalb des Gehäuses ermöglicht, wobei der T-förmige Ansatz in einer entsprechend ausgebildeten Ausnehmung zu liegen kommt und somit eine ausreichende Gewährleistung für einen sicheren Sitz der verwendeten Dichtungen vorhanden ist. Zur Verbesserung des Dichtungssitzes ist ferner vorgesehen, dass die Dichtung im Bereich des Verbindungskanals eine Verstärkung in Form eines ein- oder beidseitig einstückig angeformten Steges aufweist, der hinsichtlich der Form an die Kante der Gehäusehälfte bzw. der Druckräume angepasst ist. Das Gehäuse ist vorzugsweise zweiteilig ausgebildet und über eine klemmende Nutfederverbindung zusammengehalten ist, wobei durch die ausgebildete Dichtung und die zweiteilige Gehäuseform eine einfache Montagemöglichkeit gegeben ist. Die Zuleitungs- bzw. Ableitungsrohre können hierbei senkrecht zum Gehäuse angeordnet sein, es besteht jedoch ebenfalls die Möglichkeit, dass diese über eine Krümmung nahezu parallel zum Gehäuse angeordnet sind und somit eine zweckdienliche und kompakte Gehäuseform geschaffen werden kann.

Der besondere Vorteil der vorliegenden Erfindung besteht darin, dass im Fall des Anliegens von einem Unterdruck am Zuleitungs- oder Ableitungsrohr oder im Fall eines Überdrucks an dem Ableitungsrohr ein sicherer Stop der Infusionsflüssigkeit gewährleistet ist. Die Grundkonzeption der Erfindung beinhaltet unter anderem eine einfache Einbaumöglichkeit der Ventildichtung, insbesondere mit der Ausbildung eines T-förmigen Ansatzes, welcher in einer entsprechenden Ausnehmung der Gehäusehälften zu liegen kommt, sodass eine dauerhafte und sichere Funktion der Ventileinrichtung gewährleistet ist.

Die Erfindung wird im Weiteren anhand der Figuren näher erläutert.

Es zeigt
- Fig. 1: in einer geschnittenen Seitenansicht eine erfindungsge- mäße Ausführung der Ventileinrichtung,
- Fig. 2: eine geschnittene Seitenansicht der ersten Gehäusehälfte,
- Fig. 3: eine Draufsicht der Außenseite der Gehäusehälfte gemäß Figur 2,
- Fig. 4: eine Draufsicht der Innenseite der Gehäusehälfte gemäß Figur 2,
- Fig. 5: in einer geschnittenen Ansicht eine zweite Gehäusehälfte,
- Fig. 6: eine Draufsicht der Außenseite der Gehäusehälfte gemäß Figur 5,
- Fig. 7: eine Draufsicht der Innenseite der Gehäusehälfte gemäß Figur 5 und
- Fig. 8: eine Draufsicht und geschnittene Seitenansicht einer einzelnen Dichtung.

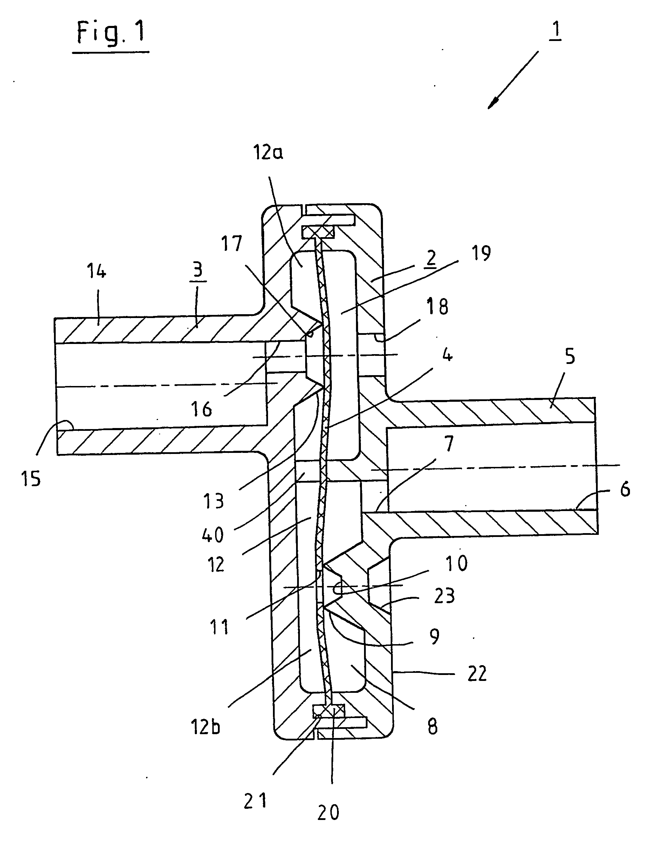

Figur 1 zeigt eine erfindungsgemäße Ventileinrichtung 1, bestehend aus einer ersten Gehäusehälfte 2 und einer zweiten Gehäusehälfte 3 sowie einer zwischen den Gehäusehälften 2, 3 einliegenden Dichtung 4 in einem zusammengebauten Zustand. Die Dichtung 4 wird hierbei zwischen den beiden Gehäusehälften 2, 3 einklemmend aufgenommen. Die erste Gehäusehälfte 2 besitzt ein Zuleitungsrohr 5 mit einem Mündungsrohr 6, welches über einen Durchbruch 7 in eine erste Vorkammer 8 der Gehäusehälfte 2 mündet. Die beispielsweise rund oder elliptisch geformte Vorkammer 8 weist eine zentrisch gelagerte Dichtringlippe 9 auf, welche einstückig aus der Gehäusehälfte 2 geformt ist und eine zwischen der Dichtringlippe 9 liegende Mulde 10 aufweist. Die an der Dichtringlippe 9 anliegende Dichtung 4 besitzt eine zentrisch zur Dichtringlippe 9 angeordnete Öffnung 11, sodass im Falle des Abhebens der Dichtung 4 von der Dichtringlippe 9 eine Verbindung durch die Öffnung 11 zum Druckraum 12 ermöglicht wird. Entsprechend der Bauform kann es sich bei dem Druckraum 12 um einen sich über die Gehäusehälften 2, 3 erstreckenden Druckraum 12 handeln oder es können zwei Druckräume 12a, 12b ausgebildet sein, die durch einen Verbindungskanal 40 miteinander verbunden sind, sodass ein identischer statischer Druck vorherrscht. Die Dichtung 4 liegt an einer zweiten Dichtringlippe 13 an, die der zweiten Gehäusehälfte 3 zugeordnet ist. Die zweite Gehäusehälfte 3 weist ein Ableitungsrohr 14 mit einem Mündungsrohr 15 auf, welches über eine Öffnung 16, in die durch die Dichtringlippe 13 gebildete Mulde 17 mündet, sodass im Fall des Abhebens der Dichtung 4 eine Öffnung zum Druckraum 12 entsteht. Gegenüber der Dichtringlippe 13 der zweiten Gehäusehälfte 3 ist in der ersten Gehäusehälfte 2 ein Durchbruch vorhanden, der eine Beaufschlagung der Dichtung 4 über eine zweite Vorkammer 19 mit Atmosphärendruck ermöglicht. Die Dichtung 4 ist zwischen den beiden Gehäusehälften 2, 3 derart eingespannt, dass im drucklosen Zustand die Dichtung 4 unmittelbar an den Dichtringlippen 9, 13 anliegt. Um ein Verrutschen der Dichtung 4 innerhalb der Gehäusehälften 2, 3 zu verhindern und somit eine sicherere Funktion der Ventileinrichtung 1 zu gewährleisten, ist im gezeigten Ausführungsbeispiel die Dichtung 4 einteilig ausgebildet und weist randseitig einen T-förmigen Ansatz 20 auf, der in einer korrespondierenden Ausnehmung 21 der ersten und zweiten Gehäusehälfte 2, 3 einliegt. Alternativ besteht die Möglichkeit eine zweiteilige Dichtung zu verwenden oder eine andere Befestigungsmöglichkeit der Dichtung 4 vorzusehen.

Durch einen statischen Druck, beispielsweise der einströmenden Infusionsflüssigkeit eines höherliegend angeordneten Vorratsbehälters in das Mündungsrohr 6 durch den Durchbruch 7 in die erste Vorkammer 8 wird die Dichtung 4 von der ersten Dichtringlippe 9 abgehoben, sodass die Infusionsflüssigkeit unter der Dichtung 4 hindurch, durch die Öffnung 11 in den Druckraum 12 einströmen kann. Aufgrund des ansteigenden Drucks in der Druckkammer 12 wird die Dichtung 4 ebenfalls im Bereich der zweiten Dichtringlippe 13 leicht angehoben, sodass die Infusionsflüssigkeit über die Mulde 17 und Öffnung 16 in das Mündungsrohr 15 einströmen kann. Bei einem Nachlassen des statischen Drucks durch die Infusionsflüssigkeit, beispielsweise einer Entleerung des Vorratsbehälters, gelangt die Dichtung 4 unmittelbar wieder zur Anlage an die Dichtringlippe 9, sodass ein Stop der Infusionsflüssigkeit gewährleistet ist. Für den Fall eines Unterdrucks an dem Mündungsrohr 6 wird darüber hinaus die Dichtung 4 an die Dichtringlippe 9 angesaugt und führt ebenfalls zu einem Stop der Strömung. Sollte dem gegenüber an dem Ableitungsrohr 14 bzw. Mündungsrohr 15 ein Unterdruck vorhanden sein, wird die Dichtung 4 ebenso angesaugt und auf die zweite Dichtringlippe 13 gepresst, sodass ein Weiterfluss gestoppt wird. Für den Fall, dass ein Überdruck an dem Ableitungsrohr 14 bzw. Mündungsrohr 15 vorherrscht, kann zwar die Dichtung 4 von der zweiten Dichtringlippe 13 abgehoben werden, jedoch wird die Dichtung 4 im Bereich der ersten Dichtringlippe 9 aufgrund des Überdrucks und der vorhandenen Vorspannung angedrückt und verhindert somit ein Einströmen der Infusionsflüssigkeit in das Mündungsrohr 6. Die Ventileinrichtung 1 ermöglicht somit ein Stop der Infusionsflüssigkeit für den Fall, dass ein Unterdruck am Zuleitungsrohr 5 oder ein Unter- oder Überdruck am Ableitungsrohr 14 vorherrscht, sodass ein Eindringen von Luft in das System des infusionsbestecks ausgeschlossen werden kann. Nur für den Fall, dass ein statischer Überdruck am Zuleitungsrohr 5 bzw. Mündungsrohr 6 anliegt, ist eine vorgegebene Strömungsrichtung des Infusionsmittels möglich.

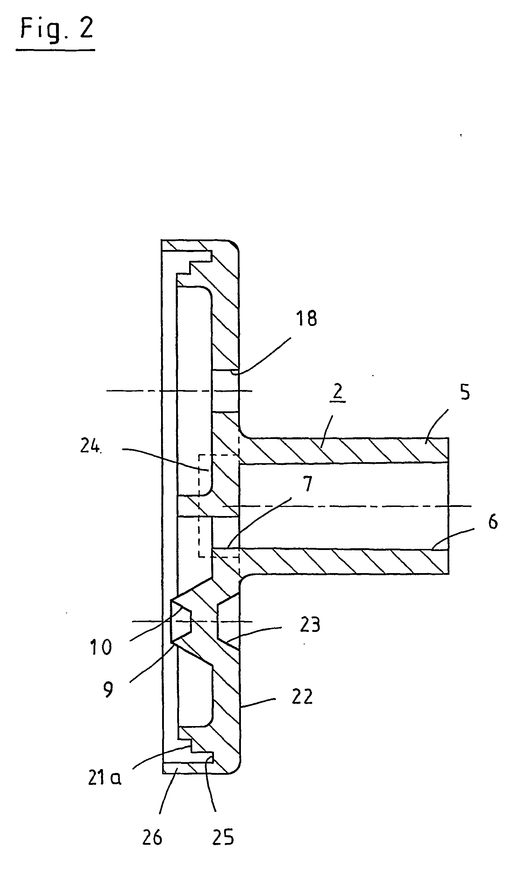

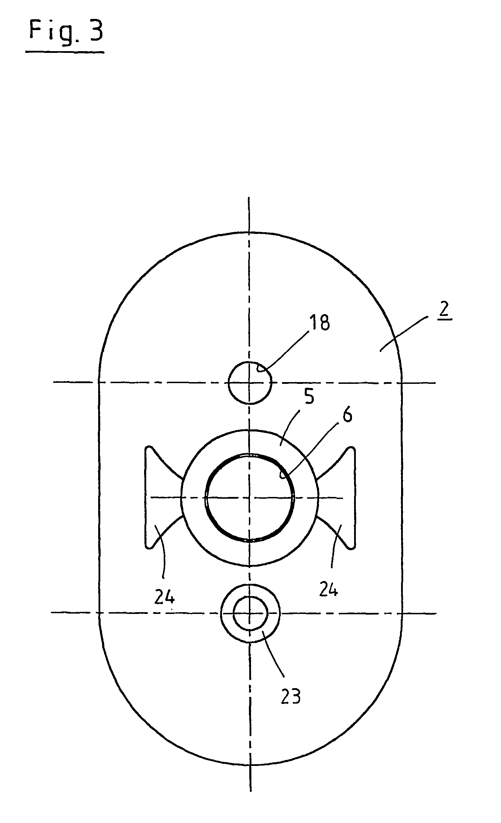

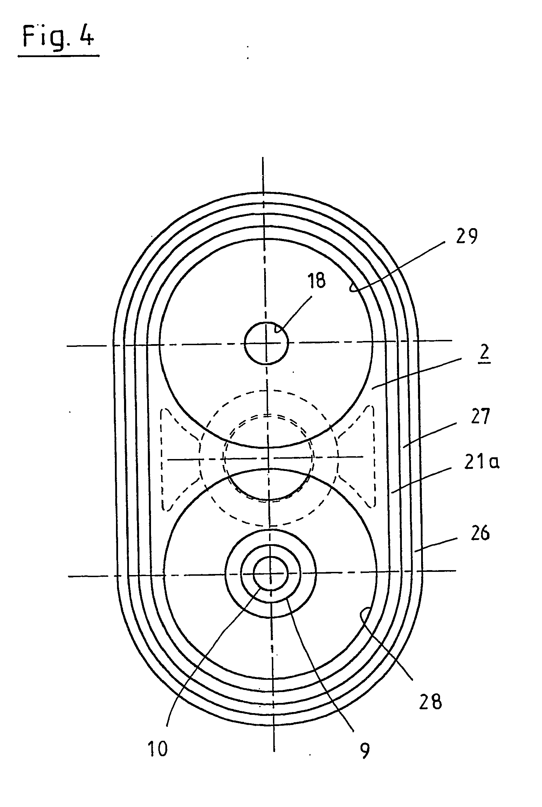

Figur 2 zeigt in einer geschnittenen Seitenansicht die erste Gehäusehälfte 2 mit ihrem Zuleitungsrohr 5 und Mündungsrohr 6 sowie die einstückig ausgeformte Dichtringlippe 9 mit ihrer Mulde 10, wie insbesondere aus Figur 3 und 4 ersichtlich. Die Gehäusehälfte 2 besitzt eine ovale Grundform, von der das Zuleitungsrohr 5 senkrecht hervorsteht. Die gesamte Gehäusehälfte 2 ist einstückig ausgebildet und weist auf ihrer Außenfläche 22 eine Mulde 23 im Bereich der ersten Dichtringlippe 9 sowie weitere dreieckförmige Mulden 24 seitlich versetzt zum Mündungsrohr 6 auf. Zur Aufnahme des T-förmigen Ansatzes 20 der Dichtung 4 ist eine Ausnehmung 21a vorhanden, welche stufenförmig in eine weitere Ausnehmung 25 übergeht. Die Ausnehmung 25 wird durch einen umlaufenden Kragen 26 begrenzt und ist zur Aufnahme eines korrespondierenden Ringansatzes der zweiten Gehäusehälfte 3 vorgesehen.

Figur 3 und 4 zeigen die erste Gehäusehälfte 2 in einer äußeren und inneren Draufsicht. Hierbei wird die Vorkammer 8 bzw. 19 durch eine ringförmige Ausnehmung 28, 29 gebildet.

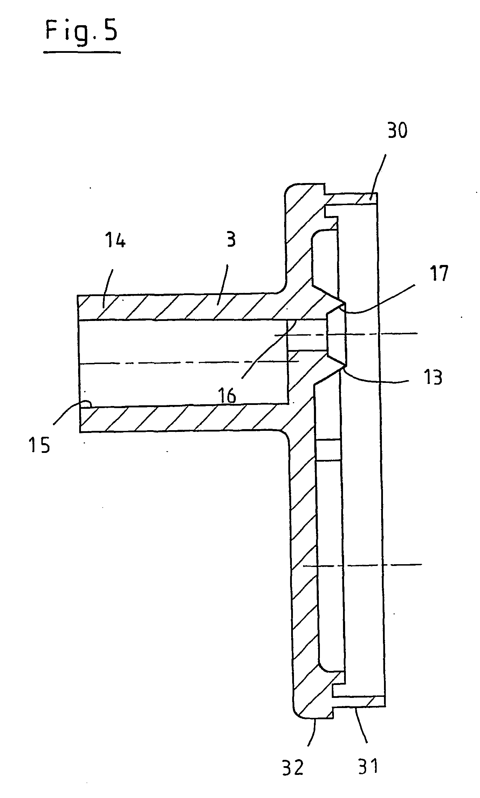

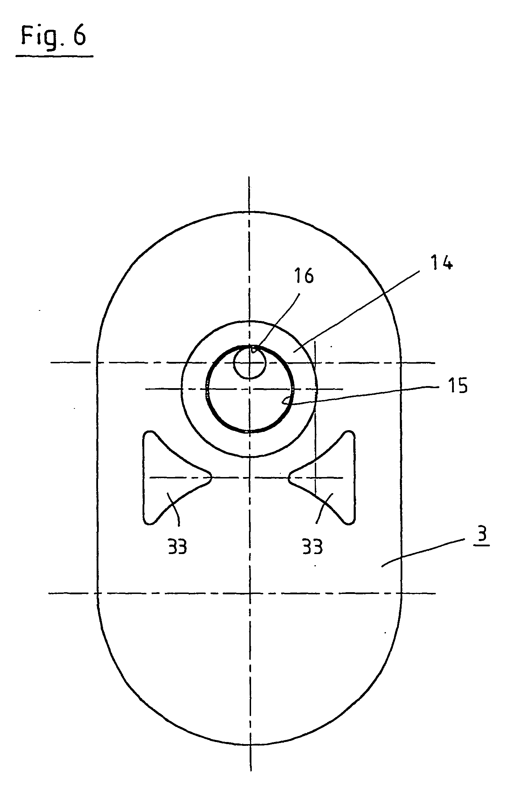

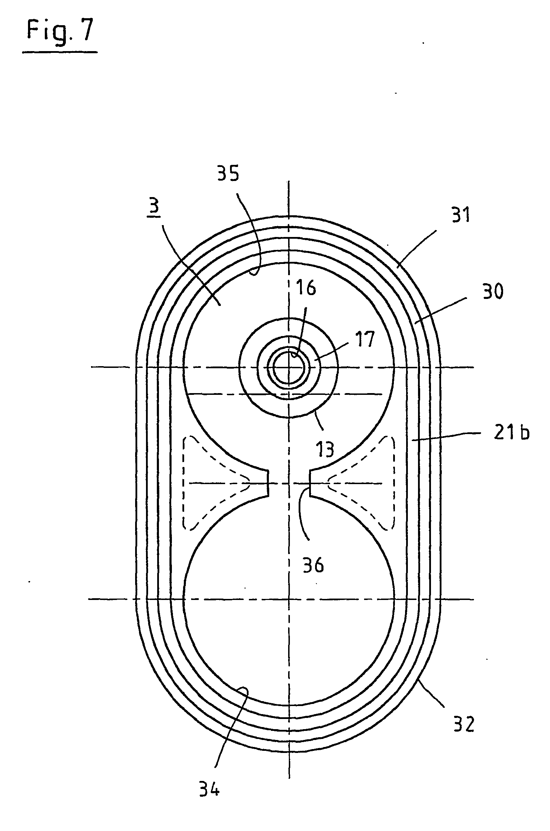

Figur 5 zeigt in einer geschnittenen Seitenansicht die zweite Gehäusehälfte 3 mit ihrem Ableitungsrohr 14 bzw. Mündungsrohr 15 und der zweiten Dichtringlippe 13 mit ihrer Mulde 17, in welche das Mündungsrohr 15 durch eine Öffnung 16 mündet. Zur Aufnahme des T-förmigen Ansatzes 20 der Dichtung 4 ist eine Ausnehmung 21 b vorgesehen, welche zusammen mit der Ausnehmung 21 a der ersten Gehäusehälfte 2 den T-förmigen Ansatz 20 aufnimmt. Die Grundform der zweiten Gehäusehälfte 3 entspricht weitesgehend der ersten Gehäusehälfte 2, wie insbesondere aus den Figuren 6 und 7 ersichtlich. Zur einklemmenden Verbindung mit der ersten Gehäusehälfte 2 weist die zweite Gehäusehälfte 3 einen umlaufenden Kragen 30 auf, der in die vorhandene Ausnehmung 25 der ersten Gehäusehälfte 2 eingreift. Der Kragen 30 wird gebildet durch einen Rücksprung 31 gegenüber der Radialfläche 32.

Figur 6 und 7 zeigen in einer Draufsicht die Innen- bzw. Außenfläche der zweitens Gehäusehälfte 3. In der äußeren Gehäusehälfte 3 sind in etwa dreieckförmige Ausnehmungen 33 ausgeformt, während auf der Innenseite der Druckraum 12 durch zwei ringförmige Ausnehmungen 34, 35 gebildet wird und die beiden Ausnehmungen 34, 35 durch eine Öffnung 36 miteinander verbunden sind.

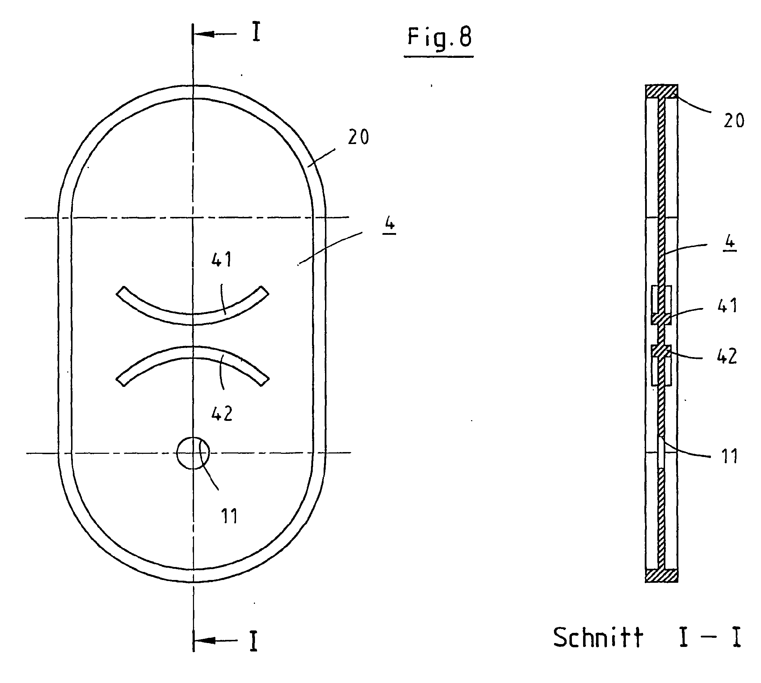

Figur 8 zeigt in einer Draufsicht und einer geschnittenen Seitenansicht eine Dichtung 4, die mit einem umlaufenden T-förmigen Ansatz 20 einstückig ausgebildet ist. Der Ansatz 20 ruht nach der Montage in einer Ausnehmung der beiden Gehäusehälften. Die vorhandenen Öffnung 11 kommt nach der Montage zentrisch innerhalb der Dichtringlippe 9 zu liegen. Zur Vermeidung einer Verspannung oder einer Deformierung der Dichtung 4 sind einstückig angeformte segmentartige Stege 41, 42 ausgebildet, die an die Form der Druckräume der beiden Gehäusehälften angepasst sind und zur Montageerleichterung dienen bzw. eine zusätzliche Abdichtung gegenüber den Gehäusehälften 2, 3 herbeiführen, sodass die Infusionsflüssigkeit nicht an der Dichtung vorbei in einen anderen Druckraum oder eine Vorkammer gelangt.

### Bezugszeichenliste

- 1: Ventileinrichtung
- 2: Gehäusehälfte
- 3: Gehäusehälfte
- 4: Dichtung
- 5: Zuleitungsrohr
- 6: Mündungsrohr
- 7: Durchbruch
- 8: Vorkammer
- 9: Dichtringlippe
- 10: Mulde
- 11: Öffnung
- 12: Druckraum
- 13: Dichtringlippe
- 14: Ableitungsrohr
- 15: Mündungsrohr
- 16: Öffnung
- 17: Mulde
- 18: Durchbruch
- 19: Vorkammer
- 20: Ansatz
- 21: Ausnehmung
- 22: Außenfläche
- 23: Mulde
- 24: Mulde
- 25: Ausnehmung
- 26: Kragen
- 27: Ringansatz
- 28: Ausnehmung
- 29: Ausnehmung
- 30: Kragen
- 31: Rücksprung
- 32: Radialfläche
- 33: Ausnehmung
- 34: Ausnehmung
- 35: Ausnehmung
- 36: Öffnung
- 40: Verbindungskanal
- 41: Steg
- 42: Steg

## Patentansprüche

1. Einweg-Ventileinrichtung (1), insbesondere Niederdruck-Rückschlagventil, zur Verwendung für ein Infusionsbesteck, mit einem eine Zuleitung (5) und eine Ableitung (14) aufweisenden Gehäuse (2, 3), wobei die Zu- und Ableitungen (5, 14) mit einem Druckraum (12) in dem Gehäuse (2, 3) verbunden sind und in dem Druckraum eine die Zuleitung (5) von der Ableitung (14) trennende Dichtung oder Membran (4) vorgesehen ist, welche unter Vorspannung gegen eine Dichtringlippe anliegt, **dadurch gekennzeichnet, dass** zwei Dichtringlippen (9, 13) nebeneinander und entgegengesetzt zueinander gerichtet vorgesehen sind, gegen welche die mindestens eine gemeinsame oder zwei getrennte Dichtung oder Membran (4) unter Vorspannung anliegt derart, dass durch einen an der Zuleitung (5) und/oder der Ableitung (14) anstehenden statischen Unterdruck oder einen Überdruck an der Ableitung (14) eine Unterbrechung des Durchflusses erfolgt.

2. Einweg-Ventileinrichtung nach Anspruch 1, **dadurch gekennzeichnet, dass** der Druckraum (12) als zwei über einen Verbindungskanal (40) miteinander verbundene Druckkammern (12a, 12b) ausgebildet ist, und dass der Verbindungskanal (40) einmal oberhalb und einmal unterhalb der Dichtung oder Membran (4) mündet.

3. Einweg-Ventileinrichtung nach Anspruch 1 oder 2, **dadurch gekennzeichnet, dass** die Zuleitung (5) in eine Vorkammer (8) mündet, in welcher die erste Dichtringlippe (9) mit einer geschlossenen Mulde (10) ausgebildet ist, an der die den Druckraum (12) begrenzende Dichtung oder Membran (4) anliegt.

4. Einweg-Ventileinrichtung nach einem der Ansprüche 1 bis 3, **dadurch gekennzeichnet, dass** die Ableitung (14) innerhalb der zweiten Dichtringlippe (13) in der Druckkammer (12) des Gehäuses (2, 3) mündet, an der die den Druckraum begrenzende Dichtung oder Membran (4) anliegt.

5. Einweg-Ventileinrichtung nach einem der Ansprüche 1 bis 4, **dadurch gekennzeichnet, dass** gegenüber der zweiten Dichtringlippe (13) eine zweite, durch die Dichtung oder Membran (4) begrenzende Vorkammer (19) vorgesehen ist, welche durch eine Gehäuseöffnung (18) mit Atmosphärendruck beaufschlagt ist.

6. Einweg-Ventileinrichtung nach einem der Ansprüche 1 bis 5, **dadurch gekennzeichnet, dass** die Dichtung oder Membran (4) im Bereich der ersten Dichtringlippe (9) eine Öffnung (16) aufweist, welche zentrisch innerhalb der ersten Dichtringlippe (9) liegend ausgebildet ist, derart, dass der Druckraum (12) gegenüber der Vorkammer (8) abgedichtet ist.

7. Einweg-Ventileinrichtung nach einem der Ansprüche 1 bis 6, dass die von der ersten und der zweiten Dichtringlippe (9, 13) eingeschlossenen Flächen kleiner als die jeweils verbleibenden Flächen der anliegenden Dichtung oder Membran (4) ausgebildet sind.

8. Einweg-Ventileinrichtung nach einem der Ansprüche 3 bis 7, **dadurch gekennzeichnet, dass** durch das Verhältnis der eingeschlossenen Flächen innerhalb der Dichtringlippen (9, 13) zu den verbleibenden Flächen der anliegenden Dichtung oder Membran (4) der Öffnungs- bzw. Schließdruck einstellbar ist.

9. Einweg-Ventileinrichtung nach einem der Ansprüche 1 bis 8, **dadurch gekennzeichnet, dass** die Dichtung oder Membran (4) am Rande mit einem einstückig ausgebildeten T-förmigen Ansatz (20) versehen ist, welcher in einer Ausnehmung (21) des Gehäuses (2, 3) angeordnet ist.

10. Einweg-Ventileinrichtung nach einem der Ansprüche 2 bis 9', **dadurch gekennzeichnet, dass** die Dichtung oder Membran (4) im Bereich des Verbindungskanals (14) mit einer Verstärkung in Form eines ein- oder beidseitig einstückig angeformten Steges versehen ist, dessen Kontur an die Kante der Gehäusehälften (2, 3) bzw. des Druckraums (12) angepasst ist.

11. Einweg-Ventileinrichtung nach einem der Ansprüche 1 bis 10, **dadurch gekennzeichnet, dass** das Gehäuse (2, 3) zweiteilig ausgebildet und über eine klemmende Nut-Feder-Verbindung zusammengehalten ist.

12. Einweg-Ventileinrichtung nach einem der Ansprüche 1 bis 11, **dadurch gekennzeichnet, dass** die Zuleitung (5) bzw. Ableitung (14) senkrecht zum Gehäuse (2, 3) und/oder parallel hierzu angeordnet sind.

## Claims

1. One-way valve device (1), in particular low-pressure non-return valve, to be used for an infusion instrument, with a housing (2, 3) having a supply line (5) and a discharge line (14), the supply and discharge lines (5, 14) being connected to a pressure space (12) in the housing (2, 3), and the pressure space having provided in it a seal or diaphragm (4) separating the supply line (5) from the discharge line (14) and bearing under prestress against a sealing-ring lip, **characterized in that** two sealing-ring lips (9, 13) are provided next to one another and in directions opposite to one another, against which the at least one joint or two separate seals or diaphragms (4) bear under prestress in such a way that an interruption in the throughflow takes place as a result of a static underpressure prevailing on the supply line (5) or the discharge line (14) or as a result of an overpressure on the discharge line (14).

2. One-way valve device according to Claim 1, **characterized in that** the pressure space (12) is designed as two pressure chambers (12a, 12b) connected to one another via a connecting duct (40), and **in that** the connecting duct (40) issues, on the one hand, above and, on the other hand, below the seal or diaphragm (4).

3. One-way valve device according to Claim 1 or 2, **characterized in that** the supply line (5) issues into a prechamber (8) in which is formed the first sealing-ring lip (9) with a closed recess (10), against which the seal or diaphragm (4) delimiting the pressure space (12) bears.

4. One-way valve device according to one of Claims 1 to 3, **characterized in that** the discharge line (14) issues, within the second sealing-ring lip (13), into the pressure chamber (12) of the housing (2, 3), against which sealing-ring lip the seal or diaphragm (4) delimiting the pressure space bears.

5. One-way valve device according to one of Claims 1 to 4, **characterized in that**, opposite the second sealing-ring lip (13), a second prechamber (19) is provided which is delimited by the seal or diaphragm (4) and which is acted upon with atmospheric pressure through a housing orifice (18).

6. One-way valve device according to one of Claims 1 to 5, **characterized in that** the seal or diaphragm (4) has, in the region of the first sealing-ring lip (9), an orifice (16) which is designed to lie centrically within the first sealing-ring lip (9), in such a way that the pressure space (12) is sealed off with respect to the prechamber (8).

7. One-way valve device according to one of Claims 1 to 6, **characterized in that** the areas enclosed by the first and the second sealing-ring lip (9, 13) are designed to be smaller than the respectively remaining areas of the seal or diaphragm (4) which comes to bear.

8. One-way valve device according to one of Claims 3 to 7, **characterized in that** the opening or closing pressure can be set by means of the ratio of the enclosed areas within the sealing-ring lips (9, 13) to the remaining areas of the seal or diaphragm (4) which comes to bear.

9. One-way valve device according to one of Claims 1 to 8, **characterized in that** the seal or diaphragm (4) is provided at the margin with a T-shaped extension (20) which is formed in one piece and which is arranged in a clearance (21) of the housing (2, 3).

10. One-way valve device according to one of Claims 2 to 9, **characterized in that** the seal or diaphragm (4) is provided, in the region of the connecting duct (14), with a reinforcement in the form of a web which is formed in one piece on one side or on both sides and the contour of which is adapted to the edge of the housing halves (2, 3) or of the pressure space (12).

11. One-way valve device according to one of Claims 1 to 10, **characterized in that** the housing (2, 3) is of two-part design and is held together via a clamping tongue-and-groove connection.

12. One-way valve device according to one of Claims 1 to 11, **characterized in that** the supply line (5) and the discharge line (14) are arranged perpendicularly to the housing (2, 3) and/or parallel thereto.

## Revendications

1. Système de soupape unidirectionnel (1), en particulier une soupape anti-retour basse pression destinée à être utilisée dans un instrument de perfusion, avec un boîtier (2, 3) présentant une arrivée (5) et une évacuation (14), moyennant quoi les arrivée et évacuation (5, 14) sont reliées à un espace de pression (12) dans le boîtier (2, 3) et un joint ou membrane (4) séparant l'arrivée (5) de l'évacuation (14) est prévu dans l'espace de pression, lequel en précontrainte repose contre une lèvre de bague d'étanchéité, **caractérisé en ce que** deux lèvres de bague d'étanchéité (9, 13) sont prévues l'une à côté de l'autre et dirigées de manière opposée l'une vers l'autre, contre lesquelles l'au moins un joint ou membrane (4) commun ou séparé en deux repose de manière à ce que par une sous-pression statique présente à l'arrivée (5) et/ou à l'évacuation (14) ou une surpression à l'évacuation (14), une interruption du débit ait lieu.

2. Système de soupape unidirectionnel selon la revendication 1, **caractérisé en ce que** l'espace de pression (12) est formé comme deux chambres de pression (12a, 12b) reliées entre elles par un canal de liaison (40) et que le canal de liaison (40) débouche une fois au-dessus et une fois au-dessous du joint ou de la membrane (4).

3. Système de soupape unidirectionnel selon la revendication 1 ou 2, **caractérisé en ce que** l'arrivée (5) débouche dans une préchambre (8) dans laquelle la première lèvre de bague d'étanchéité (9) est formée avec une cavité (10) fermée sur laquelle le joint ou membrane (4) délimitant l'espace de pression (12) repose.

4. Système de soupape unidirectionnel selon l'une des revendications 1 à 3, **caractérisé en ce que** l'évacuation (14) débouche à l'intérieur de la deuxième lèvre de bague d'étanchéité (13) dans la chambre de pression (12) du boîtier (2, 3) sur laquelle le joint ou membrane (4) délimitant l'espace de pression repose.

5. Système de soupape unidirectionnel selon l'une des revendications 1 à 4, **caractérisé en ce qu'**opposée à la deuxième lèvre de bague d'étanchéité (13), une deuxième préchambre (19) délimitée par le joint ou membrane (4) est prévue, laquelle est alimentée en pression atmosphérique par une ouverture de boîtier (18).

6. Système de soupape unidirectionnel selon l'une des revendications 1 à 5, **caractérisé en ce que** le joint ou membrane (4) présente au niveau de la première lèvre de bague d'étanchéité (9) une ouverture (16) qui est formée de manière à se trouver centrée à l'intérieur de la première lèvre de bague d'étanchéité (9), de telle manière que l'espace de pression (12) soit rendu étanche par rapport à la préchambre (8).

7. Système de soupape unidirectionnel selon l'une des revendications 1 à 6, **caractérisé en ce que** les surfaces incluses par les première et deuxième lèvres de bague d'étanchéité (9, 13) sont formées comme les surfaces restantes respectives du joint ou membrane (4) en contact.

8. Système de soupape unidirectionnel selon l'une des revendications 3 à 7, **caractérisé en ce que** par le rapport des surfaces incluses à l'intérieur des lèvres de bague d'étanchéité (9, 13) aux surfaces restantes du joint ou membrane (4) en contact, la pression d'ouverture respectivement de fermeture est réglable.

9. Système de soupape unidirectionnel selon l'une des revendications 1 à 8, **caractérisé en ce que** le joint ou membrane (4) est pourvu sur son rebord d'un embout (20) monobloc en forme de T, lequel est placé dans un évidement (21) du boîtier (2, 3) .

10. Système de soupape unidirectionnel selon l'une des revendications 2 à 9, **caractérisé en ce que** le joint ou membrane (4) est pourvu au niveau du canal de liaison (14) d'un renforcement sous la forme d'une branche formée sur un côté ou sur les deux de manière monobloc dont le contour est adapté au bord des moitiés de boîtier (2, 3) respectivement de l'espace de pression (12).

11. Système de soupape unidirectionnel selon l'une des revendications 1 à 10, **caractérisé en ce que** le boîtier (2, 3) est formé de deux parties et est solidarisé par un assemblage serré à rainure et languette.

12. Système de soupape unidirectionnel selon l'une des revendications 1 à 11, **caractérisé en ce que** l'arrivée (5) respectivement l'évacuation (14) est placée de manière perpendiculaire au boîtier (2, 3) et/ou parallèlement à celui-ci.
